# EUROPÄISCHE PATENTSCHRIFT

(11) **EP 1 158 966 B1**
(45) Veröffentlichungstag und Bekanntmachung des Hinweises auf die Patenterteilung: **11.06.2003**
(21) Anmeldenummer: 00912549.3
(22) Anmeldetag: 03.03.2000
(51) Int. Cl.: A61K 9/50

(54) **BELADENE BAKTERIENGHOSTS ALS TRÄGER- UND TARGETINGVEHIKEL**
LOADED BACTERIAL GHOSTS AS CARRIER AND TARGETING VEHICLES
HOTES BACTERIENS CHARGES UTILISES COMME EXCIPIENTS DE SUPPORT ET DE CIBLAGE

(30) Priorität: 05.03.1999 DE 19909770
(43) Veröffentlichungstag der Anmeldung: 05.12.2001
(73) Patentinhaber: Lubitz, Werner, Dr., 1080 Wien (AT)
(72) Erfinder: LUBITZ, Werner, A-1080 Wien (AT); HUTER, Veronika, A-2544 Leobersdorf (AT)
(74) Vertreter: Dey, Michael, Dr.
(86) Internationale Anmeldenummer: EP0001906
(87) Internationale Veröffentlichungsnummer: WO00053163

(56) Entgegenhaltungen:
- EP-A- 0 242 135
- DE-A- 2 541 685
- DE-A- 3 919 644
- M.P. SZOSTAK ET AL.: "Bacterial ghosts: non-living candidate vaccines" JOURNAL OF BIOTECHNOLOGY, Bd. 44, Nr. 1, 26. Januar 1996 (1996-01-26), Seiten 161-170, XP004036862 Amsterdam (NL)
- W. LUBITZ ET AL.: "Extended recombinant bacterial ghost system" JOURNAL OF BIOTECHNOLOGY, Bd. 73, Nr. 2-3, 20. August 1999 (1999-08-20), Seiten 261-273, XP002138570 Amsterdam (NL)
- V. HUTER ET AL.: "bacterial ghosts as drug carrier and targeting vehicles" JOURNAL OF CONTROLLED RELEASE, Bd. 61, Nr. 1-2, 27. August 1999 (1999-08-27), Seiten 51-63, XP000907214 Amsterdam (NL)

## Beschreibung

Die Erfindung betrifft die Verwendung von Bakterienghosts als Träger- und Targetingvehikel für eingekapselte Substanzen, z.B. Wirkstoffe.

Leere Bakterienhüllen, sogenannte Bakterienghosts, können durch kontrollierte, heterologe Expression eines Gens, das eine partielle Lyse der Zellmembran bewirkt, in gram-negativen Bakterien hergestellt werden (EP-A-0 291 021). Ein Beispiel für ein derartiges lytisches Gen ist das Gen E des Bakteriophagen PhiX174, das für ein Polypeptid kodiert, welches in den Zellwandkomplex gram-negativer Bakterien inseriert und durch die Oligomerisierung zur Ausbildung einer transmembranen Tunnelstruktur durch die innere und äußere Membran führt. Der innere Durchmesser dieser Tunnelstruktur kann je nach Lysebedingungen 40 bis 200 nm oder 500 bis 1000 nm betragen. Durch diesen Tunnel wird das cytoplasmatische Material der Zelle freigesetzt und eine leere Zellhülle mit intakter Morphologie zurückgelassen. Die Verwendung von Bakterienghosts als Totimpfstoffe oder Adjuvanzien sowie die Herstellung rekombinanter Bakterienghosts, die in ihrer Membran heterologe Oberflächenproteine tragen, wird in WO 91/13555 und WO 93/01791 beschrieben.

Weiterhin können Ghosts auch aus Gram-positiven Bakterien durch Verwendung eines chimären E-L-Lysegens hergestellt werden (US-A-5,075,223).

Überraschenderweise wurde festgestellt, daß Bakterienghosts hervorragend als Träger oder Targetingvehikel für Wirkstoffe geeignet sind. Ein erster Vorteil der Bakterienghosts besteht darin, daß die Applikation ohne weiteres über den natürlichen Infektionsweg von Pathogenen wie etwa über den Respirations- oder den Gastrointestinaltrakt möglich ist. Darüber hinaus bietet das Applikationssystem von Wirkstoffen über Ghosts als Träger aufgrund der Spezifität der Bakterienghosts für verschiedene Gewebearten ein wirkungsvolles Targeting. Damit wird der Wirkstoff an den gewünschten Zielort, z.B. den entsprechenden potenziellen Infektionsort der Ausgangsbakterien, mit hoher Effizienz gebracht. Dieser Vorteil von natürlichen Hüllen pathogener Bakterien als Vektoren kann bei anderen Applikationsformen, wie z.B. Liposomen, mit eingebauten äußeren Membranproteinen nur mühsam und unzulänglich erreicht werden.

Da Bakterienghosts herstellbar sind, die ausschließlich den gewünschten Wirkstoff enthalten, kann ein hoher Beladungsgrad und somit eine hohe Effizienz des Wirkstoffs erreicht werden. Zudem stellen Ghosts ein sicheres Trägermaterial dar, da es sich nicht um lebensfähige Organismen handelt. Schließlich handelt es sich bei Ghosts um Produkte mit einer hohen immunstimulatorischen Wirkung aufgrund des Vorhandenseins von Lipopolysacchariden und Peptidoglykanen, so daß keine zusätzlichen Adjuvanzien zugegeben werden müssen, da die Ghosts den Adjuvanzeffekt per se erfüllen.

Ein Gegenstand der Erfindung ist somit die Verwendung von Bakterienghosts zur Verpackung von Wirkstoffen.

Ein weiterer Gegenstand der Erfindung ist die Verwendung von Bakterienghosts als Träger- oder/und Targetingvehikel für einen Wirkstoff.

Der Wirkstoff kann jeder beliebige, in das Innere der Bakterienghosts transportierbare und dort vorzugsweise immobilisierbare Wirkstoff sein. Vorzugsweise wird der Wirkstoff ausgewählt aus pharmakologisch wirksamen Substanzen und Markierungssubstanzen. Beispiele für pharmakologisch wirksame Substanzen sind Polypeptide wie etwa Antikörper, therapeutisch wirksame Polypeptide wie Zytokine, Interferone, Chemokine etc., Enzyme und immunogene Polypeptide oder Peptide. Ein weiteres Beispiel für Wirkstoffe sind Nukleinsäuren, insbesondere therapeutische Nukleinsäuren, z.B. Nukleinsäuren für die Gentherapie, die vorzugsweise in Form eines chromosomal integrierbaren Vektors vorliegen, oder Nukleinsäuren für eine Nukleinsäure-Vakzinierung, Antisense-Nukleinsäuren oder Ribozyme. Noch weitere Beispiele für Wirkstoffe sind niedermolekulare Wirksubstanzen, Peptide, Hormone, Antibiotika, Antitumormittel, Steroide, Immunmodulatoren etc. Die Wirkstoffe können in den Bakterienghosts in gelöster Form, als Suspensionen oder/und als Emulsionen gegebenenfalls in Kombination mit geeigneten Träger- oder/und Hilfsstoffen vorliegen. Weiterhin können die Wirkstoffe auch diagnostische Markierungssubstanzen, z.B. fluoreszierende Substanzen, Farbstoffe oder Röntgenkontrastmittel sein.

Auch nichtmedizinische Wirkstoffe können in Ghosts verpackt werden, z.B. Wirkstoffe aus dem Agrarbereich wie Insektizide, Herbizide, Mittel gegen Nematoden, Enzyme zur Bodenverbesserung, Dünger, Wachstumsförderer, wasserbindende Proteine zur besseren Durchfeuchtung oder Wasserbindung in der Atmosphäre. Andere Anwendungen sind die Verpackung von Farbstoffen für die Druckindustrie, z.B. fälschungssichere Tinten mit immunologischer Nachweismöglichkeit und die Verpackung von Vitaminen oder Probiotika für die Lebensmittelindustrie. Ebenso möglich ist die Verpackung von kosmetischen Mitteln oder von Stoffen wie Salzen oder anderen ionischen Substanzen.

Vorzugsweise liegt der Wirkstoff in den Bakterienghosts in immobilisierter Form vor, d.h. daß der verpackte Wirkstoff unter physiologischen Bedingungen eine ausreichende Zeitdauer innerhalb der Bakterienghosts verbleibt, um einen Transport zur Zielzelle bzw. zum Zielgewebe zu ermöglichen. Die Immobilisierung des Wirkstoffs kann mittels kovalenter oder nichtkovalenter Wechselwirkungen, z.B. elektrostatischer Wechselwirkungen, hochaffiner biologischer Wechselwirkungen, durch mechanische Retention oder eine Kombination von zwei oder mehreren der genannten Möglichkeiten erfolgen.

In einer bevorzugten Ausführungsform der Erfindung erfolgt die Immobilisierung des Wirkstoffs über direkte oder indirekte Wechselwirkungen mit einem Rezeptor, der auf der Membraninnenseite, z. B. der Innenseite der Zytoplasmamembran, der Ghosts, als integraler Membranbestandteil oder als nichtintegraler Membranbestandteil auf der Membran verankert lokalisiert ist. Der Rezeptor kann beispielsweise ein heterologes Polypeptid sein, das über einen oder mehrere Membrananker in die zytoplasmatische Membran der Ghosts integriert ist und durch heterologe Expression entsprechender Fusionsproteine, die mindestens eine Membranankerdomäne sowie mindestens eine Rezeptordomäne enthalten, in den Bakterienzellen vor der Lyse zu den Ghosts erzeugt wird. Bevorzugte Beispiele für Rezeptordomänen sind Avidin oder Streptavidin, die zur Ausbildung hochaffiner Bindungen mit Biotin oder Biotinanaloga in der Lage sind. Besonders bevorzugt ist Streptavidin. Die Verankerung von Streptavidin in Bakterienghosts erfolgt vorzugsweise durch rekombinante Expression eines Streptavidin-Fusionsproteins mit einem C-terminalen Membrananker in der zytoplasmatischen Membran von Bakterien vor der zur Bildung von Ghosts führenden Lyse. Darüber hinaus sind auch andere Rezeptordomänen geeignet, z.B. Antikörperbindungsstellen, Lectine etc., die mit einem Bindepartner eine hochaffine Bindung eingehen können.

Alternativ ist eine Verankerung des Rezeptors auch erst nach Lyse der Ghosts an der Membraninnenseite möglich, beispielsweise durch Verwendung eines Rezeptors mit zwei Bindungsstellen, wobei eine Bindungsstelle in der Lage ist, an natürliche oder rekombinante Strukturen auf der Membraninnenseite mit hoher Affinität zu binden, und die zweite Bindungsstelle für die direkte oder indirekte Immobilisierung von Wirkstoffen zur Verfügung steht.

Ein auf der Membraninnenseite der Ghosts lokalisiertes Rezeptormolekül kann eine direkte oder indirekte Immobilisierung von Wirkstoffen im Inneren der Ghosts bewirken. Bei einer direkten Immobilisierung wird ein Rezeptor gewählt, der mit dem in die Ghosts zu verpackenden Wirkstoff eine ausreichend starke Wechselwirkung eingehen kann, um eine weitgehende oder vollständige Retention des Wirkstoffs im Ghostinneren zu bewirken. Hierzu kann man beispielsweise mit Biotin, Haptenen oder/und Zuckern modifizierte Wirkstoffe einsetzen, die mit Rezeptoren wie Streptavidin, Antikörpern oder Lectinen eine stabile Bindung eingehen können. Vorzugsweise verwendet man einen modifizierten Wirkstoff, der eine oder mehrere Biotingruppen trägt und mit hoher Affinität an einen Streptavidin-Rezeptor binden kann.

Alternativ kann auch eine indirekte Immobilisierung des Wirkstoffs an den Rezeptor erfolgen, die z.B. über Wirkstoff-bindende Substanzen, die weiterhin mindestens eine zusätzliche Bindungsstelle für den Rezeptor besitzen, vermittelt wird. Beispiele für solche Wirkstoff-bindende Substanzen sind Polymere, z.B. Proteine wie Polylysin oder Polysaccharide wie etwa Protaminsulfat oder Dextran. Die Wirkstoff-bindenden Substanzen tragen weiterhin Rezeptorbindungsgruppen, z.B. Biotin oder Biotinanaloga, Haptene oder an Lectine bindefähige Zuckergruppen, die in der Lage sind, eine Verankerung an den auf der Membran lokalisierten Rezeptor zu bewirken.

Die Herstellung der erfindungsgemäßen mit Wirkstoffen beladenen Ghosts gemäß dem zuvor genannten Aspekt der Erfindung umfaßt zunächst die Herstellung der Bakterienghosts nach bekannten Methoden, z.B. durch Transformation der Bakterienzelle mit einem Lysegen, vorzugsweise dem Gen E des Phagen PhiX174 oder dem chimären E-L-Gen. Die Expression des Lysegens in der Bakterienzelle erfolgt vorzugsweise durch eine regulierbare Expressionskontrollsequenz, z.B. durch das Temperatur-regulierbare Promotor/Repressor-System λ-pR/cl857. Bei diesem Expressionskontrollsystem werden die transformierten Bakterien bei Temperaturen unterhalb 30°C kultiviert. Durch Temperaturerhöhung, vorzugsweise auf ≥ 40 °C wird der thermosensitive λcl857 Repressor inaktiviert und das Lysegen exprimiert, was zur Ausbildung einer transmembranen Tunnelstruktur in der Zellhülle führt, wobei die Zellen innerhalb von wenigen Minuten lysiert werden. Durch mutierte λ-Promotor/Operator-Systeme ist eine Anzucht der Bakterien auch bei höheren oder tieferen Temperaturen, z.B. 37°C möglich (WO98/07874). Die Bakterienghosts können dann durch Zentrifugation geerntet und nach Waschen und gegebenenfalls Gefriertrocknen zur Beladung mit Wirkstoffen eingesetzt werden. Hierzu werden die Ghosts mit einer den zu verpackenden Wirkstoff enthaltenden Lösung oder/und Suspension in Kontakt gebracht, unter Bedingungen, die das Eindringen ausreichender Wirkstoffmengen in die Bakterienghosts erlauben. Sofern erforderlich, werden darüber hinaus Rezeptorsubstanzen zugesetzt, die eine Immobilisierung der Wirkstoffmoleküle an der Membraninnenseite der Ghosts ermöglichen. Die Zugabe der Rezeptormoleküle kann vor, gleichzeitig oder nach dem Inkontaktbringen der Ghosts mit dem zu verpackenden Wirkstoff erfolgen.

Alternativ oder/und zusätzlich kann die Immobilisierung des Wirkstoffs über die Bildung einer Matrix im Ghostinneren erfolgen. Diese Matrix ist vorzugsweise eine Polymermatrix, die im Ghostinneren in situ entsteht und das Herausdiffundieren von Wirkstoffen aus den Ghosts verhindert. Die Polymermatrix kann durch Polymerisation oder/und Copolymerisation geeigneter Monomere oder/und durch Zusammenlagerung aggregierbarer Substanzen im Inneren der Bakterienghosts erzeugt werden. Die Polymerisation kann durch Einstellung entsprechender Bedingungen, z.B. durch Temperaturerhöhung, UV-Strahlung oder/und Zugabe geeigneter Initiatoren gestartet werden. Zweckmäßigerweise werden physiologisch verträgliche Monomere wie etwa Hydroxyfettsäuren, Aminosäuren, Saccharide oder Derivate davon verwendet, die zur Bildung eines im Körper unter physiologischen Bedingungen abbaubaren Polymeren führen.

Besonders bevorzugt wird die Matrix durch eine enzymkatalysierte Polymerbildung erzeugt. Hierzu werden geeignete Enzyme auf der Innenwand der Bakterienghosts immobilisiert, beispielsweise durch Integration in die Innenwand der zytoplasmatischen Membran (wie für Streptavidin beschrieben) oder indirekt, z.B. durch Bindung biotinylierter Enzyme an Streptavidinmoleküle auf der Ghostinnenmembran. Beispielsweise kann man zu diesem Zweck Enzyme verwenden, welche die Synthese von Polyhydroxyfettsäuren, z.B. Polyhydroxybuttersäure wie etwa die PHB-Synthase, Polysacchariden wie etwa Glucosyltransferasen oder Polypeptiden wie etwa nichtribosomale Polypeptid-synthetisierende Enzyme, die in der Peptidoglykansynthese vorkommen, katalysieren. Die durch Zugabe geeigneter Monomere und gegebenenfalls biochemischer Energieäquivalente wie etwa ATP in Gegenwart der Enzym erfolgende Wirkstoffe führt dazu, daß die sich im Ghostinneren befindenden Wirkstoffe in der durch die Enzyme gebildeten Matrix eingesponnen und somit im Ghostinneren gehalten werden.

Alternativ kann die Matrix auch durch Zusammenlagerung von aggregierbaren Substanzen, z.B. von Molekülen oder kolloidalen Partikeln erfolgen. Diese Zusammenlagerung kann durch Änderung der Umgebungsbedingungen, z.B. Temperaturänderung oder/und pH-Änderung initiiert werden.

Bei derjenigen Ausführungsform der Erfindung, in der die Wirkstoffe durch Bindung einer Matrix im Ghostinneren immobilisiert werden, werden zunächst die zu verkapselnden Wirkstoffen in die Ghosts eingebracht und anschließend die Matrix gebildet.

Die erfindungsgemäßen Ghosts mit darin eingekapselten Wirkstoffen sind hervorragend als Träger- und Targetingvehikel geeignet, da die Ghosts schon aufgrund ihrer Natur als Bakterienhüllen bereits bevorzugt sich an bestimmte Zelltypen anlagern bzw. von Zellen des Immunsystems aufgenommen werden. Verbessert werden kann dieses Targeting noch durch Verwendung von Ghosts mit modifizierten Hüllen, d.h. Ghosts, die targetspezifische, d.h. für Zielzellen oder Zielgewebe spezifische Oberflächenmoleküle an ihrer Membranaußenseite tragen. Die Einführung dieser targetspezifischen Moleküle wie etwa Zucker, z.B. Mannose oder Fucose, oder Invasin aus Yersinien oder Invasinderivaten, kann durch rekombinante Expression entsprechender Fusionspolypeptide in der Bakterienzelle vor der Lyse oder/und durch Anlagerung mittels eines geeigneten Rezeptorsystems (z.B. Streptavidin/Biotin) erfolgen.

Eine Ausführungsform der Erfindung ist der Einsatz der Wirkstoffe enthaltenden Ghosts für medizinische Zwecke. Die Verabreichung von Wirkstoffen, z.B. pharmakologischen Wirkstoffen, Antigenen, Antikörpern oder Nukleinsäuren, über Ghosts ist zur Prävention oder/und zur Bekämpfung aller Arten von Erkrankungen geeignet, z.B. zur Bekämpfung von durch Pathogene wie Viren, Bakterien, Parasiten oder Pilzen hervorgerufenen Erkrankungen, oder zur Prävention oder/und zur Bekämpfung von Tumor- oder Autoimmunerkrankungen oder zur Gentherapie. Als Wirkstoff wird dabei eine gegen die jeweilige Erkrankung wirksame Substanz verwendet, die nach Transport und gegebenenfalls Internalisierung in der Zielzelle ihre physiologische Wirkung hervorrufen. Die vorliegende Erfindung ermöglicht auch die Verabreichung von Wirkstoffkombinationen, d.h. die Ghosts können mehrere verschiedene Wirkstoffe enthalten oder es können Mischungen von Ghosts mit jeweils verschiedenen Wirkstoffen verwendet werden. Weiterhin kann die Verabreichung von Wirkstoffen über Ghosts auch für diagnostische Zwecke (Imaging) eingesetzt werden.

Eine besonders bevorzugte Anwendung ist der Einsatz von Bakterienghosts als Träger- und Targetingvehikel für die Gentherapie. Durch Verpackung von Nukleinsäuren wie DNA oder RNA in Ghosts kann die mangelnde Spezifität bestehender Nukleinsäure-Vehikel wie z.B. Liposomen entscheidend verbessert werden. Der Vorteil von Bakterienghosts als Trägervehikel besteht weiterhin darin, daß sie eine hohe Kapazität für die Beladung mit Nukleinsäuren besitzen. Sie sind zudem als Vektoren ungefährlich, da sie keine lebenden Zellhüllen darstellen. Für gentherapeutische Anwendungen können die Nukleinsäuren in den Ghosts beispielsweise mit Polyhydroxyalkanoaten, z.B. Polyhydroxybuttersäure oder Copolymeren von Hydroxybuttersäure mit anderen Hydroxyfettsäuren wie etwa 3-Hydroxytridecansäure komplexiert werden. Dabei können die Nukleinsäuren in einer wachsenden Polymermatrix immobilisiert werden oder Komplexe aus Nukleinsäuren und amorphen Polyhydroxyfettsäuregranula hergestellt werden. Noch eine weitere Möglichkeit zur Verkapselung von Nukleinsäure in Bakterienghosts besteht darin, DNA-bindende Proteine wie etwa Polylysin oder Protamine, bei denen es sich um globuläre, stark alkalische Proteine mit relativ geringem Molekulargewicht zwischen 1000 und 5000 handelt, zu verwenden. Protamine können aus entfetteten Vogel- oder Fischspermien durch Schütteln mit verdünnten Säuren in Form von kristallinen Salzen, z.B. Protaminsulfate gewonnen werden. Protamine können in Ghosts in Form von Fusionsproteinen in die Membran eingelagert werden oder alternativ durch Biotinylierung an membrangebundenes Streptavidin in den Ghosts verankert werden.

Noch eine besonders bevorzugte Anwendung ist der Einsatz von Bakterienghosts zur Herstellung einer Nukleinsäure-Vakzine, insbesondere zur Herstellung einer DNA-Vakzine, und der Einsatz von Bakterienghosts als Träger- oder/und Targetingvehikel für eine Nukleinsäure-Vakzine, insbesondere für eine DNA-Vakzine.

Bakterienghosts als Träger- oder Targetingvehikel zur Nukleinsäure-Vakzinierung führen zur Bildung einer wirkungsvollen und langandauerenden spezifischen Immunantwort. Die die Nukleinsäuren enthaltenden Bakterienghosts werden von primären Antigen-präsentierenden Zellen (APC), wie etwa dendritischen Zellen und Makrophagen durch spezifische Rezeptoren aufgenommen und in antigene Peptide fragmentiert. Zusätzlich wird mit hoher Effizienz das Antigen, welches durch die verpackte DNA-Sequenz kodiert wird, in den APC exprimiert. Dies hat zur Folge, daß das Antigen auf der Oberfläche der APC im Kontext mit MHC-I oder/und MHC-II Strukturen den T-Lymphozyten präsentiert wird und eine Immunantwort induzieren kann. Untersuchungen ergaben in diesem Zusammenhang, daß Antigenprozessierungen und Präsentation durch MHC-I und II-Komplexe erfolgen, wobei eine humorale und zelluläre Immunantwort induziert wird, wie sie auch bei bakteriellen Infektionen mit Lebendkeimen beobachtet wird.

Die in die Bakterienghosts verpackte Nukleinsäure ist vorzugsweise in einer im Empfängerorganismus nicht replizierbaren Form. Sie enthält eine Sequenz, die für das in der Zielzelle zu exprimierende Antigen kodiert, in expressionsfähiger Form, d.h. in operativer Verknüpfung mit in der Zielzelle aktiven Expressionskontrollsequenzen wie etwa Promotoren und gegebenenfalls Enhancern, um eine hohe Genexpression zu erlauben, Polyadenylierungssequenzen, um eine korrekte Termination der transkribierten mRNA zu gewährleisten, oder/und Translationsinitiations-Sequenzen, um eine hohe Proteinproduktion zu ermöglichen. Weiterhin können die Nukleinsäuren einen bakteriellen Replikationsursprung, welcher die Amplifikation großer Nukleinsäuremengen in Bakterien wie etwa E.coli erlaubt, ein prokaryontisches Selektionsmarkergen, z.B. für eine Antibiotikaresistenz, ein Reportergen, das eine einfache Bestimmung der Expressionshöhe ermöglicht, z.B. das GFP-Gen oder/und immunmodulatorische Sequenzen enthalten.

Vorzugsweise ist die Nukleinsäure eine DNA, besonders bevorzugt eine Plasmid-DNA, die in zirkulärer oder/und in linearer Form vorliegen kann. Es ist jedoch auch die Verwendung von RNA-Vakzinen oder Vakzinen auf Basis von Nukleinsäureanaloga, die transkribierbar sind, aber eine erhöhte physiologische Stabilität aufweisen, denkbar.

Der die Expression der Antigen-kodierenden Sequenz antreibende Promotor ist vorzugsweise ein starker viraler Promotor/Enhancer, z.B. der Rous Sarcoma Virus (RSV)-Promotor/Enhancer, der Murine Leukemia Virus (MLV)-Promotor/Enhancer, der SV40-Promotor/Enhancer und besonders bevorzugt der Cytomegalovirus (CMV)-Promotor/Enhancer. Als Transkriptionsterminatoren können die Polyadenylierungssequenzen aus SV40 oder aus dem Rinderwachstumshormongen, vorzugsweise jedoch aus dem Kaninchen-β-Globin-Gen verwendet werden.

Als Antigen wird dabei ein mit der jeweiligen Erkrankung assoziiertes Polypeptid oder ein Peptidfragment davon verwendet, das nach Expression in der Zielzelle eine Immunantwort induziert. Die vorliegende Erfindung ermöglicht auch die Verabreichung von Kombinationsvakzinen, d.h. die Ghosts können mehrere verschiedene Antigen-kodierende Nukleinsäuren enthalten, die z.B. vom selben Erreger oder von unterschiedlichen Erregern stammen können, oder es können Mischungen von Ghosts mit jeweils verschiedenen Antigen-kodierenden Nukleinsäuren verwendet werden.

In einer Ausführungsform der Erfindung können sogenannte homologe Kombinationen von Bakterienghost und Antigen-kodierender Nukleinsäure verwendet werden, wobei z.B. der Bakterienghost Oberflächenstrukturen trägt,die aus der gleichen Spezies oder dem gleichen Organismus stammen wie das von der Nukleinsäure-Vakzine kodierte Antigen. Gegebenenfalls kann der Ghost auf seiner Oberfläche sogar eine dem kodierten Antigen entsprechende Oberflächenstruktur tragen. Diese homologe Ghost/Nukleinsäure-Kombination kommt insbesondere zur Vakzinierung gegen bakterielle Infektionen in Frage, sie kann jedoch - bei Verwendung von rekombinanten Ghosts mit entsprechenden Oberflächenstrukturen - auch auf die Vakzinierung gegen andere Erkrankungen, z.B. virale Erkrankungen, erweitert werden.

Alternativ dazu wird eine heterologe Ghost/Nukleinsäure-Kombination verwendet. Bei einer derartigen heterologen Kombination erfüllt der Bakterienghost im allgemeinen Adjuvansfunktionen. Es sind jedoch auch Ausführungsformen möglich, bei denen ein aus einem pathogenen Bakterium stammender Ghost in Kombination mit einer heterologen Nukleinsäure als Kombinationsvakzine gegen zwei unterschiedliche Erreger verwendet wird.

Schließlich sind die Bakterienghosts auch als Träger- oder Targetingvehikel für den Agrarbereich geeignet, wo sie zum Ausbringen von Wirkstoffen wie etwa Herbiziden, Fungiziden oder/und Insektiziden verwendet werden können.

Besonders bevorzugt stammen die Ghosts von gram-negativen Bakterien, die z.B. ausgewählt werden aus Eschericha coli, Klebsiella, Salmonella, Enterobacter, Pseudomonas, Vibrio, Actinobacillus, Haemophillus, Pasteurella, Bordetella, Helicobacter, Francisella, Brambamella, Erwinia, Pantoea, Streptomyces, Frankia, Serratia, Agrobacterium, Azotobacter, Bradyrhizobium, Burkholderia, Rhizobium, Rhizomonas und Sphingomonas. Besonders bevorzugte Beispiele für gram-positive Bakterien sind Staphylococcus, Streptococcus und Bacillus.

Die pharmazeutische Verabreichung der Wirkstoff-enthaltenden Ghosts kann nach geläufigen Methoden erfolgen, beispielsweise oral, aerogen, z.B. intranasal, intraokulär, topisch oder parenteral, z.B. intramuskulär, intraperitonial, intravenös oder subkutan.

Die Verabreichung der Ghosts erfolgt vorzugsweise auf demselben Weg, wie auch eine natürliche Infektion des Organismus mit dem Erreger erfolgt. So können Bakterienghosts mit Wirkstoffen, die zur Bekämpfung von pathogenen Erregern vorgesehen sind, deren Hautpeintrittspforte der Gastrointestinaltrakt ist (E.coli, Salmonella, Vibrio oder Helicobacter), oral verabreicht werden. Ghosts aus Erregern von Lungenentzündungen, die entsprechende Wirkstoffe enthalten, z.B. Actinobacillus, Pasteurella, Pseudomonas oder Haemophilus, werden vorzugsweise aerogen verabreicht.

Die erfindungsgemäße Verabreichung von Bakterienghosts mit Wirkstoffen ist nicht nur für die Humanmedizin, sondern auch für die Tiermedizin, insbesondere für die protektive Impfung von Haustieren, wie etwa Schweinen, Kühen etc. geeignet.

Für landwirtschaftliche Anwendungsformen können die Ghosts über den Boden, die Luft oder als Kapseln an Samen verabreicht werden.

Die Applikation von Wirkstoffen über Bakterienghosts hat gegenüber bisherigen Applikationsformen eine Vielzahl von Vorteilen. So reichen bereits geringe Wirkstoffmengen zur Erzielung einer starken Wirkung aus. Weiterhin ist eine Zielzellen/Gewebe-spezifische Verabreichung der Wirkstoffe möglich. Aufgrund der bereits für sich immunogen wirkenden Bakterienghosthüllen wird eine Adjuvanswirkung erzielt. Der in den Ghost eingeschlossene Wirkstoff ist vor Abbau durch physiologische Prozesse, z.B. durch Enzyme wie Proteasen, Nukleasen oder Hydrolasen geschützt. Darüber hinaus ist eine Kombination mit weiteren Wirkstoffen möglich. Schließlich können die Bakterienghosts kostengünstig hergestellt und die Wirkstoffe einfach und kostengünstig formuliert werden.

Noch ein weiterer Gegenstand der Erfindung sind Bakterienghosts mit einem darin verkapselten Wirkstoff, wobei es sich bei dem Wirkstoff beispielsweise um eine Nukleinsäure handeln kann.

Schließlich betrifft die Erfindung auch eine pharmazeutische oder landwirtschaftliche Zusammensetzung, umfassend einen Bakterienghostmit einem darin verpackten Wirkstoff. Die erfindungsgemäße pharmazeutische Zusammensetzung kann in Form üblicher pharmazeutischer Präparate vorliegen, z.B. als injizierbare oder aerogen applizierbare Lösung oder Suspension, als orales Präparat, z.B. als Tablette, Kapsel oder Dragee, als Creme oder Salbe etc. Weiterhin kann die Zusammensetzung als rekonstituierbares Lyophilisat vorliegen.

Die erfindungsgemäße Zusammensetzung ist erhältlich durch ein Verfahren umfassend die Schritte:
(a) Bereitstellen von Bakterienghosts und
(b) Inkontaktbringen der Bakterienghosts mit einem Wirkstoff unter Bedingungen, die zu einer Verpackung und vorzugsweise zu einer Immobilisierung des Wirkstoffs in den Ghosts führen.

Weiterhin wird die Erfindung durch die nachfolgenden Abbildungen und Beispiele erläutert. Es zeigen:
- Figur 1: eine schematische Darstellung des Streptavidin-Verankerungsplasmids pAV1, das ein Fusionsgen E'-FXa-StrpA unter Kontrolle des induzierbaren lac-Promotors (lacPO), den Replikationsursprung ColE1 und das Ampicillinresistenzgen bla enthält.
- Figur 2: die Reaktionskinetik von in Steptavidin-Ghosts gebundener Alkalischer Phosphatase.

### Beispiele

### 1. Material und Methoden

### 1.1 Herstellung von Streptavidin-kodierenden Plasmiden

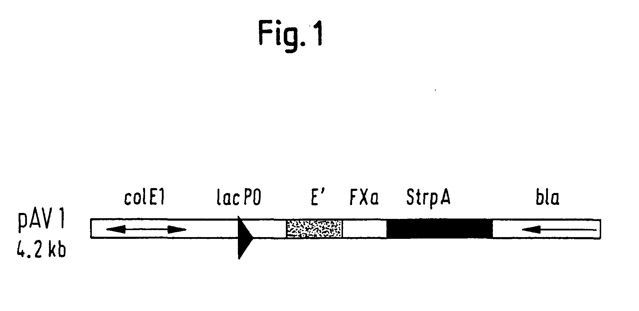

Das Plasmid pBGG9 (British Biotechnology Limited) wurde mit den Restriktionsenzymen Ndel und HindIII geschnitten. Ein das vollständige Streptavidingen (Argarana et al., Nucleic Acids Res. 14 (1986), 1871-1882) enthaltendes 495 bp DNA-Fragment wurde durch Agarose-Gelelektrophorese und anschließende Elektroelution isoliert. Die Ndel-Restriktionsstelle wurde mit Klenow Polymerase aufgefüllt, und das Fragment wurde zwischen die HincII- und HindIII Restriktionsstellen von M13K11RX (Waye et al., Nucleic Acids Res. 13 (1985), 8561-8571) inseriert. Das resultierende Phageimid M13FN enthält 160 Kodons des Streptavidingens fusioniert an das 3'-Ende einer kurzen Sequenz, welche für die Erkennungssequenz lle-Glu-Gly-Arg des Proteasefaktors Xa (FXa) kodiert. Diese FXa-StrpA Kassette wurde durch Restriktionsspaltung mit BamHI isoliert, und das resultierende 509 bp lange DNA-Fragment wurde in das mit BamHI linearisierten Plasmid pSK- (Stratagene, Cleveland, Ohio) subkloniert, um das Plasmid pFN6 zu erzeugen. Dasselbe 509 bp lange BamHI-Fragment wurde in den mit BamHI gespaltenen Membrantargetingvektor pKSEL5 inseriert, wobei das Plasmid pAV1I mit dem Streptavidingen fusioniert an die 5'-terminale Membranankersequenz E' erhalten wurde (Fig. 1).

### 1.2 Herstellung von Streptavidinghosts

E.coli NM522-Zellen (Stratagene) wurden simultan mit dem Lyseplasmid pML1 (Szostak et al., J. Biotechnol. 44 (1996), 161-170) und dem Streptavidin-kodierenden Plasmid pAV1 transformiert. Die Transformanten wurden in LB-Medium (10 g/l Trypton, 5 g/l Hefeextrakt, 5 g/l NaCI) mit Ampicillin (200 µg/ml) und Kanamycin (50 µg/ml) bei 28°C kultiviert. 1 I Medium wurde mit einer Übernachtkultur inokuliert, die aus einer einzelnen Transformantenkolonie stammte, und als Vorkultur für einen Fermenter (Typ MRD 60TE, Meredos GmbH, Bovenden, Deutschland) verwendet. Die Bakterien wurden im Fermenter in einem Volumen von 10 I unter Belüftung und Rühren bis zum Erreichen einer optischen Dichte bei 600 nm von 0,4 kultiviert. Dann wurde IPTG auf eine Endkonzentration von 3 mM zur Induktion der Expression von Streptavidin zugegeben. Nach 30 min wurden 0,2 M MgSO₄ zugegeben und 20 min danach wurde die Expression des Lyseproteins E durch Temperaturerhöhung von 28°C auf 42°C induziert. Nach 1 h wurden die Zellen durch Zentrifugation bei 4000 g geerntet. Die Resuspension der Pellets in destilliertem Wasser (Endvolumen 5 I) führte zu einer sofortigen Lyse. Die Ghosts wurden zweimal in einem großen Volumen von Tris-gepufferter Salzlösung (TBS) gewaschen und anschließend lyophilisiert.

### 1.3 Licht- und Elektronenmikroskopie

Lichtmikroskopische Untersuchungen wurden mit einem Olympus AX70 True Research System Microscope durchgeführt. Transmissions-Elektronenmikrographien wurden mit einem Siemens Elmiscop 101 Elektronenmikroskop aufgenommen. Raster-Elektronenmikrographien wurden mit einem Hitachi S-800 Feldemissions-Raster-Elektronenmikroskop aufgenommen. Die Fixierung von Zellen und die Vorbereitung für die Elektronenmikroskopie erfolgte wie bei Witte et al. (J. Bacteriol. 172 (1990), 4109-4114) beschrieben.

Zum Nachweis von Streptavidin wurden die Ghosts mit goldmarkiertem Albumin-Biotin (10 nm, Sigma Immunochemicals) verdünnt in Tris-Puffer (10 mM Tris, 150 mM NaCI pH 7,4) unter Schütteln bei 37°C für 20 min inkubiert, gewaschen und für die Elektronenmikroskopie fixiert.

### 1.4 SDS-Polyacrylamid-Gelelektrophorese und Westernblot

Ghosts oder Proteinproben wurden in einem Gelbeladepuffer (2% SDS, 5 % 2-Mercaptoethanol, 10% Glycerin und 0,003 % Bromphenolblau in 0,063 M Tris-HCI Puffer pH 6,8) für 5 min aufgekocht und auf 10 % SDS Polyacrylamidgel nach der Methode von Laemmli (Nature 227 (1970), 680 bis 685) aufgetrennt. Westernblots wurden wie von Towbin et al. (Biotechnology 24 (1992), 145-149) beschrieben durchgeführt. Die Blots wurden in TBS mit 1% Rinderserum blockiert und mit Anti-Streptavidin-Antiserum aus Kaninchen (Sigma Immunochemicals) inkubiert.

### 1.5 Bindung von biotinylierter Alkalischer Phosphatase und Fluoreszenz-markiertem Biotin

Biotinylierte Alkalische Phosphatase (Pierce) wurde 1:1000 in Tris-Puffer verdünnt. 2 mg lyophilisierte Ghosts wurden in 500 µl verdünnter Lösung von Alkalischer Phosphatase suspendiert und unter Schütteln bei 37°C für 30 min inkubiert. Die Proben wurden bei 10 000 g zentrifugiert und dreimal in 20 ml Trispuffer und ein viertes Mal in Diethanolaminpuffer (10 mM Diethanolamin, 0,5 mM MgCl₂ pH 9,5) gewaschen und anschließend in 6 Aliquots unterteilt. Dann wurde Substrat (2,5 mM p-Nitrophenylphosphat in Diethanolaminpuffer) zugegeben und die Reaktionen nach 0,5, 1, 2, 4, 8 oder 16 min durch Zugabe eines gleichen Volumens an 7 M NaOH gestoppt. Die Proben wurden bei 10 000 g zentrifugiert und die Überstände bei 410 nm vermessen.

Es wurde ein molarer Adsorptionskoeffizient ε = 18,5x10³xM⁻¹xcm⁻¹ bestimmt und zur Berechnung der gebildeten Menge an p-Nitrophenol nach der Lambert-Beer Gleichung verwendet. Die Anzahl der pro Ghost gebundenen Moleküle Alkalischer Phosphatase wurde unter der Annahme berechnet, daß eine Aktitivätseinheit der Alkalischen Phosphatase der Freisetzung von 1 µmol Nitrophenol pro min bei pH 9,5 und 37°C entspricht. Eine Einheit Alkalische Phosphatase entspricht 0,7 µg; das Molekulargewicht ist 140 000 und 1 mg Ghost enthalten 6,7x10⁸ einzelne Hüllen.

Zur Bindung von Fluoreszenz-markiertem Biotin (FITC-Biotin) wurden die Ghosts wiederholt in PBS gewaschen, bis die relative Fluoreszenzintensität im Überstand geringer als 0,5 bei 530 nm (Anregung bei 490 nm) war. 1 mg lyophilisierte Ghosts wurden in 2 ml einer Lösung mit 0,4056 µg FITC-Biotin/100 ml TBS unter Schütteln für 30 min inkubiert. Die Proben wurden bei 10 000 g zentrifugiert und die Fluoreszenzintensität in den Überständen gemessen.

### 1.6 Biotinylierung von Polylysin

Poly-L-Lysin-Hydrobromid, Molekulargewicht 18 000 (Sigma) wurde unter Verwendung des folgenden Protokolls biotinyliert: 6 mg Polylysin wurden in 1 ml phosphatgepufferter Salzlösung (PBS) aufgenommen. 50 µl einer Lösung von 640 µg Biotin-N-Hydroxysuccinimidester (Boehringer Mannheim) in 200 µl DMSO wurden zugegeben und der pH-Wert unter Verwendung von 0,5 M NaOH auf 10 eingestellt. Der Reaktionsansatz wurde über Nacht bei Raumtemperatur gerührt und anschließend für 48 h gegen Wasser dialysiert. Ein HABA-Test (Sigma) ergab ein Bindeverhältnis von 2 mol Biotin pro mol Polylysin.

### 1.7 Fluoreszenzmarkierung von DNA

Ein beliebiges ausgewähltes Plasmid (pUC18) wurde zur Erzeugung von fluoreszenzmarkierter DNA verwendet. Die Markierung erfolgte unter Verwendung der Polymerase Kettenreaktion und markierter Nukleotide (Cy3-dCTP, Pierce). Die Reaktionsansätze enthielten 200 µM dATP, 200 µM dGTP, 200 µm dTTP, 200 µM dCTP (davon 75% Cy3-dCTP), jeweils 1 µM Oligonukleotidprimer, 0,2 ng/µl linearisierte Plasmid DNA und 0,02 U/µl Taq DNA Polymerase in Polymerasepuffer. Das Reaktionsprotokoll war wie folgt: Vordenaturierung 4 min bei 95°C; 35 Cyclen: 1 min 95°C/1 min 60°C/3 min 72°C; 5 min Schlußextension bei 72°C. Die Proben wurden phenolisiert, mit Ethanol präzipitiert, in 10 mM Tris HCI pH 8,0 resuspendiert und bei -20°C aufbewahrt.

### 1.8 Bindung von fluoreszenzmarkiertem Dextran und DNA/Polylysin

1 mg lyophilisierte Streptavidin-Ghosts wurden in 1 ml Tris Puffer suspendiert. 50 µl einer wässrigen Lösung (1 mg/ml) von biotinyliertem Fluoreszenz-markiertem Dextran (Molecular Probes Europe BV) wurden zugegeben und die Mischung unter Schütteln bei 37°C für 1 h inkubiert. Die Ghosts wurden dreimal in 1,5 ml Tris-Puffer gewaschen und durch Lichtmikroskopie analysiert. Zur Bildung von Komplexen aus fluoreszenzmarkierter DNA und biotinyliertem Polylysin wurden verdünnte Lösungen von DNA (0,1 µg/µl in HBS [150 mM NaCI, 20 mM HEPES pH 7,3-]) und Poly-L-Lysin (1 µg/µl in HBS) hergestellt. Die Lösungen wurden in einem Gewichtsverhältnis DNA/Polylysin von 10:1 vereinigt und schnell vermischt. Dann wurden Streptavidin-Ghosts darin suspendiert, unter Schütteln bei 37°C für 1 h inkubiert, gewaschen und lichtmikroskopisch analysiert.

### 2. Ergebnisse

### 2.1 Membranverankerung von Streptavidin

Bei Verwendung von Bakterienghosts als Vehikel zum Transport von Wirkstoffen sollte der Wirkstoff innerhalb der Bakterienhülle fixiert werden. Rekombinante Ghosts, die Streptavidin verankert in ihrer Hülle enthalten, sind in der Lage biotinylierte Verbindungen mit hoher Affinität zu binden. Hierzu wurde - wie unter 1.1 beschrieben - das Plasmid pAV1 hergestellt, welches ein Hybridgen enthält, das aus den 54 5'-terminalen Kodons des Gens E des Bakteriophagen PhiX174 (E') gefolgt von einer in frame-Fusion der FXa-StrpA-Kassette besteht. Dieses Plasmid ist schematisch in Fig. 1 dargestellt.

### 2.2 Herstellung von Streptavidin-Ghosts

Es stehen mehrere E-spezifische Lyseplasmide mit verschiedenen Gen E Expressionskontrollsequenzen, Replikationsursprüngen und Selektionsmarkergenen zur Verfügung (Szostak et al., J. Biotechnol. 44 (1996), 161-170). Das hier verwendete Plasmid pML1 enthält das Gen E unter transkriptioneller Kontrolle des λp_{R}-cl857 Systems. Der Beginn der Lyse kann im E.coli Stamm NM522/pML1 durch Abnahme der optischen Dichte bei 600 nm etwa 10 min nach Erhöhung der Kultivierungstemperatur von 28°C auf 42°C beobachtet werden.

Zur Herstellung von Ghosts durch ein alternatives E-Lyseprotokoll wurden 0,2 M MgSO₄ zum Kulturmedium 20 min vor der Induktion der Gen E Expression gegeben. Bei dieser Prozedur wird das Protein E in den bakteriellen Zellwallkomplex eingebaut, aber die Zell-Lyse wird durch die hohe Salzkonzentration im umgebenden Medium gehemmt. In mit MgSO₄ behandelten Kulturen wird die Expression des Gens E für 1 h durchgeführt und die Zellen werden anschließend durch Zentrifugation geerntet. Eine Resuspension dieser Zellen in Wasser oder Puffer mit geringer lonenstärker führt zu einer sofortigen, explosiven Lyse, die erheblich größere Lyselöcher als die normale E Lyse erzeugt.

### 2.3 Mikroskopische Darstellung von durch alternative Lyse erzeugten Ghosts

Ghosts sind von lebenden Zellen durch lichtmikroskopische Untersuchungen unterscheidbar, bei denen sie deutlich transparenter als intakte Bakterien erscheinen. Lichtmikroskopische Untersuchungen an Ghosts, die durch alternative E-Lyse erzeugt worden waren, zeigten Zellen mit abgesprengten Polkappen oder Rissen in der Mitte, die sie in zwei Hälfte öffnen. Die Ghosts erschienen etwas verlängert.

### 2.4 Nachweis von Streptavidin mit Gold-konjugiertem Biotin

Zum Nachweis der Lokalisierung von Streptavidin in den Ghosts, wurden Streptavidin-Ghosts mit goldmarkierten Albumin-Biotinpartikeln inkubiert, gewaschen und elektronenmikroskopisch untersucht. Ultradünnschnitte zeigten Goldpartikel, die ausschließlich entlang der Ghost-Innenmembran angeordnet waren.

### 2.5 Bestimmung der Streptavidinverankerung in Ghosts

Zur Bestimmung ihres Streptavidingehalts wurden Streptavidin-Ghosts zusammen mit definierten Mengen an reinem Streptavidin als Kontrolle durch SDS-Polyacrylamidgelelektorphorese analysiert. Die Gele wurden auf Nitrozellulosemembranen transferiert und mit Anti-Streptavidin-Antiserum behandelt. Eine densitometrische Analyse der Streptavidin-spezifischen Banden auf Westernblots zeigte einen Streptavidingehalt von etwa 5% des gesamten Zellgewichts.

### 2.6 Funktionelle Bindung von biotinylierter alkalischer Phosphatase und FITC Biotin und Quantifizierung der Bindestellen

Zur Bestimmung der Biotinbindekapazität von Streptavidin-Ghosts wurde ein enzymatischer Test entwickelt. Streptavidin-Ghosts und Streptavidinnegative Ghosts (Ghosts ohne auf ihrer Membran verankertes Streptavidin), die beide durch das alternative Lyseprotokoll hergestellt worden waren, und Streptavidin-Ghosts, die durch Standardlyse hergestellt worden waren, wurden mit biotinylierter Alkalischer Phosphatase inkubiert. Nach gründlichem Waschen wurde die Menge an zurückgehaltenem Enzym unter Verwendung von p-Nitrophenylnitrophosphat als Substrat bestimmt. Während in Streptavidin-negativen Ghostproben praktisch keine Reaktion sichtbar war, zeigten alternativ lysierte Streptavidinghosts eine hellgelbe Färbung. Die Reaktion wurde nach kontrollierten Intervallen gestoppt und die Absorption der Probenüberstände bei 410 nm gemessen.

Nach der unter 1.5 angegebenen Berechnungsmethode wurde die Anzahl der pro Ghost gebundenen Moleküle Alkalischer Phosphatase mit etwa 200 bestimmt. Interessanterweise waren die durch normale Lyse erzeugten Streptavidin-Ghosts negativ im enzymatischen Test. Folglich ist bei großen Wirkstoffmolekülen wie der Alkalischen Phosphatase des Vorhandenseins von durch das alternative Lyseprotokoll erzeugten größeren Löchern in der Zellmembran erforderlich, um eine effiziente Diffusion in das Innere des Ghosts zu ermöglichen.

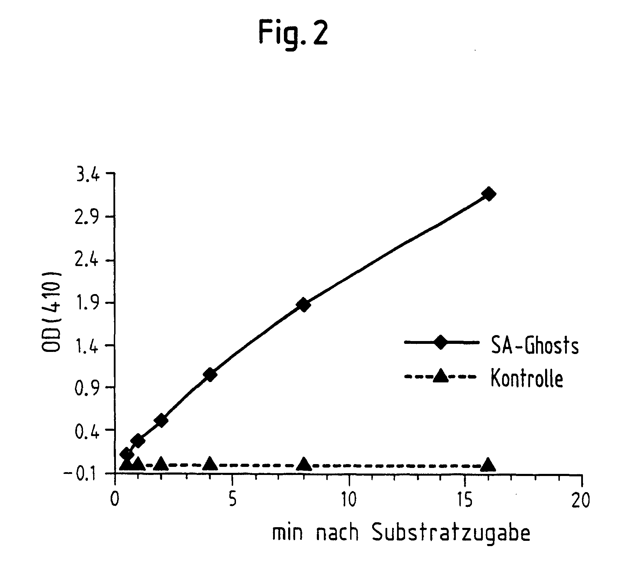

Fig. 2 zeigt die Kinetik der Reaktion von biotinylierter Alkalischer Phosphatase, die an durch alternative Lyse erzeugten Streptavidin-Ghosts gebunden wurde. Als Kontrolle wurden Streptavidin negative Ghosts erzeugt durch alternative Lyse verwendet.

Ein entsprechender Test wurde unter Verwendung von fluoreszenzmarkiertem Biotin durchgeführt. Ghost- und Streptavidin-Ghost-Proben wurden mit FITC-Biotin inkubiert, zentrifugiert und die restliche Fluoreszenz von ungebundener Markierung in den Überständen gemessen. Diese viel kleineren Moleküle (Molekulargewicht 832) wurden in einer Anzahl von 2060 ± 400 pro Ghost gebunden.

### 2.7 Bindung von fluoreszenzmarkiertem biotinyliertem Dextran und fluoreszenzmarkierter DNA

Fluoreszenzmarkiertes biotinyliertes Dextran und fluoreszenzmarkierte DNA wurden als Modell verwendet, um die Fixierung von Verbindungen zu zeigen, die zum Targeting von Wirkstoffen in Streptavidin-Ghosts verwendet werden können. Hierzu wurden Streptavidin-Ghosts mit einer Mischung von biotinyliertem Poly-L-Lysin und fluoreszenzmarkierter DNA bzw. mit fluoreszenzmarkiertem biotinyliertem Dextran inkubiert und durch Fluoreszenzlichtmikroskopie analysiert. In beiden Fällen konnte die Fluoreszenzmarkierung auf den Ghosts nachgewiesen werden. Negative Kontrollen (Ghosts ohne Streptavidin) wurden nicht angefärbt.

## Patentansprüche

1. Verfahren zur Herstellung eines wirkstoffhaltigen Bakterienghosts, umfassend die Schritte
(a) Bereitstellen von Bakterienghosts und
(b) Inkontaktbringen der Bakterienghosts mit einem Wirkstoff.

2. Bakterienghost mit einem darin verkapselten Wirkstoff erhältlich mit dem Verfahren nach Anspruch 1.

3. Bakterienghost nach Anspruch 2,
**dadurch gekennzeichnet,**
**dass** der Wirkstoff eine Nukleinsäure ist.

4. Verwendung von Bakterienghosts nach Anspruch 2 oder 3 zur Verpackung von Wirkstoffen.

5. Verwendung von Bakterienghosts nach Anspruch 2 oder 3 als Trägeroder/und Targetingvehikel für einen Wirkstoff.

6. Verwendung nach Anspruch 4 oder 5,
**dadurch gekennzeichnet,**
**dass** der Wirkstoff ausgewählt ist aus pharmakologisch wirksamen Substanzen, Markierungssubstanzen, landwirtschaftlich wirksamen Substanzen und Farbstoffen.

7. Verwendung nach einem der Ansprüche 4 bis 6,
**dadurch gekennzeichnet,**
**dass** der Wirkstoff in den Bakterienghosts in immobilisierter Form vorliegt.

8. Verwendung nach Anspruch 7,
**dadurch gekennzeichnet,**
**dass** die Immobilisierung über Wechselwirkungen mit einem Rezeptor erfolgt, der auf der Membraninnenseite der Ghosts lokalisiert ist.

9. Verwendung nach Anspruch 8,
**dadurch gekennzeichnet,**
**dass** der Rezeptor ein heterologes Polypeptid ist, das in die zytoplasmatische Membran der Ghosts integriert ist.

10. Verwendung nach Anspruch 8 oder 9,
**dadurch gekennzeichnet,**
**dass** das heterologe Polypeptid ein Streptavidin oder Avidin enthaltendes Fusionspolypeptid ist.

11. Verwendung nach einem der Ansprüche 7 bis 10,
**dadurch gekennzeichnet,**
**dass** eine direkte Immobilisierung des Wirkstoffs an den Rezeptor erfolgt.

12. Verwendung nach Anspruch 11,
**dadurch gekennzeichnet,**
**dass** man einen mit Rezeptorbindungsgruppen derivatisierten Wirkstoff verwendet.

13. Verwendung nach einem der Ansprüche 7 bis 10,
**dadurch gekennzeichnet,**
**dass** eine indirekte Immoblisierung des Wirkstoffs an den Rezeptor erfolgt.

14. Verwendung nach Anspruch 13,
**dadurch gekennzeichnet,**
**dass** die indirekte Immobilisierung des Wirkstoffs an den Rezeptor über wirkstoffbindende Substanzen erfolgt, die weiterhin mindestens eine zusätzliche Bindungsstelle für den Rezeptor besitzen.

15. Verwendung nach Anspruch 14,
**dadurch gekennzeichnet,**
**dass** die wirkstoffbindenden Substanzen ausgewählt werden aus Polylysin, Dextran und Protaminsulfat.

16. Verwendung nach Anspruch 7,
**dadurch gekennzeichnet,**
**dass** die Immobilisierung über die Bildung einer Matrix im Ghostinneren erfolgt.

17. Verwendung nach Anspruch 16,
**dadurch gekennzeichnet,**
**dass** die Matrix durch Polymerisation oder/und Copolymerisation von Monomeren im Ghostinneren entsteht.

18. Verwendung nach Anspruch 16 oder 17,
**dadurch gekennzeichnet,**
**dass** die Polymerisation durch Temperaturerhöhung, durch UV-Strahlung oder/und Zugabe von Initiatoren gestartet wird.

19. Verwendung nach Anspruch 16 oder 17,
**dadurch gekennzeichnet,**
**dass** eine enzymkatalysierte Polymerisation erfolgt.

20. Verwendung nach Anspruch 19,
**dadurch gekennzeichnet,**
**dass** Enzyme eingesetzt werden, die die Synthese von Polyhydroxyfettsäuren, Polysacchariden oder Polypeptiden katalysieren.

21. Verwendung nach Anspruch 16,
**dadurch gekennzeichnet,**
**dass** die Matrix durch Zusammenlagerung von aggregierbaren Substanzen gebildet wird.

22. Verwendung nach einem der Ansprüche 4 bis 21,
**dadurch gekennzeichnet,**
**dass** die Ghosts heterologe, für Zielzellen oder Zielgewebe spezifische Oberflächenmoleküle enthalten.

23. Verwendung nach einem der Ansprüche 4 bis 22 zur Herstellung eines Mittels für den medizinischen Bereich.

24. Verwendung nach Anspruch 23 zur Herstellung eines Mittels zur Prävention oder/und zur Bekämpfung von durch Pathogene hervorgerufenen Erkrankungen, von Tumorerkrankungen oder von Autoimmunerkrankungen.

25. Verwendung nach Anspruch 23 oder 24 zur Herstellung eines Mittels für die Gentherapie.

26. Verwendung nach Anspruch 23 oder 24 zur Herstellung eines Mittels für die Nukleinsäure-Vakzinierung.

27. Verwendung nach Anspruch 23 zur Herstellung eines Mittels für diagnostische Zwecke.

28. Verwendung nach einem der Ansprüche 23 bis 27 zur Herstellung eines Mittels, wobei die Verabreichung der Ghosts auf demselben Weg wie die natürliche Infektion des Organismus mit dem Erreger vorgesehen ist.

29. Verwendung nach einem der Ansprüche 4 bis 22 im Agrarbereich.

30. Verwendung nach einem der Ansprüche 4 bis 29,
**dadurch gekennzeichnet,**
**dass** die Ghosts mehrere verschiedene Wirkstoffe enthalten.

31. Verwendung nach einem der Ansprüche 4 bis 30,
**dadurch gekennzeichnet,**
**dass** Mischungen von Ghosts mit jeweils verschiedenen Wirkstoffen eingesetzt werden.

32. Verwendung nach einem der Ansprüche 4 bis 30,
**dadurch gekennzeichnet,**
**dass** die Ghosts von gram-negativen oder/und gram-positiven Bakterien stammen.

33. Verwendung von Bakterienghosts zur Herstellung einer Nukleinsäure-Vakzine.

34. Verwendung von Bakterienghosts als Träger- oder/und Targetingvehikel für eine Nukleinsäure-Vakzine.

35. Verwendung nach Anspruch 33 oder 34,
**dadurch gekennzeichnet,**
**dass** die in den Ghosts verpackte Nukleinsäure eine für das zu exprimierende Antigen kodierende Sequenz in operativer Verknüpfung mit Expressionskontrollsequenzen enthält.

36. Verwendung nach Anspruch 35,
**dadurch gekennzeichnet,**
**dass** die Nukleinsäure weiterhin einen bakteriellen Replikationsursprung, ein prokaryontisches Selektrionsmarkergen, ein Reportergen oder/und immunmodulatorische Sequenzen enthält.

37. Verwendung nach einem der Ansprüche 33 bis 36,
**dadurch gekennzeichnet,**
**dass** die Ghosts mehrere verschiedene Antigen-kodierende Nukleinsäuren enthalten.

38. Verwendung nach einem der Ansprüche 33 bis 37,
**dadurch gekennzeichnet,**
**dass** man eine homologe Kombination von Bakterienghosts und Antigen-kodierende Nukleinsäuren verwendet.

39. Verwendung nach einem der Ansprüche 33 bis 37,
**dadurch gekennzeichnet,**
**dass** man eine heterologe Kombination von Bakterienghosts und Antigen-kodierenden Nukleinsäuren verwendet.

40. Pharmazeutische oder landwirtschaftliche Zusammensetzung, umfassend einen Bakterienghost nach Anspruch 2 oder 3 mit einem darin verpackten Wirkstoff.

## Claims

1. Process for producing a bacterial ghost containing an active substance comprising the steps
(a) providing bacterial ghosts and
(b) contacting the bacterial ghosts with an active substance.

2. Bacterial ghost containing an active substance encapsulated therein obtainable by the process as claimed in claim 1.

3. Bacterial ghost as claimed in claim 2,
**characterized in that**
the active substance is a nucleic acid.

4. Use of bacterial ghosts as claimed in claim 2 or 3 to package active substances.

5. Use of bacterial ghosts as claimed in claim 2 or 3 as carrier or/and targeting vehicles for an active substance.

6. Use as claimed in claim 4 or 5,
**characterized in that**
the active substance is selected from pharmacologically active substances, labelling substances, substances that are effective in agriculture and dyes.

7. Use as claimed in one of the claims 4 to 6,
**characterized in that**
the active substance is present in the bacterial ghosts in an immobilized form.

8. Use as claimed in claim 7,
**characterized in that**
the immobilization is achieved by means of interactions with a receptor which is located on the inner side of the ghost membrane.

9. Use as claimed in claim 8,
**characterized in that**
the receptor is a heterologous polypeptide which is integrated into the cytoplasmic membrane of the ghosts.

10. Use as claimed in claim 8 or 9,
**characterized in that**
the heterologous polypeptide is a fusion polypeptide containing streptavidin or avidin.

11. Use as claimed in one of the claims 7 to 10,
**characterized in that**
the active substance is directly immobilized on the receptor.

12. Use as claimed in claim 11,
**characterized in that**
an active substance derivatized with receptor binding groups is used.

13. Use as claimed in one of the claims 7 to 10,
**characterized in that**
the active substance is indirectly immobilized on the receptor.

14. Use as claimed in claim 13,
**characterized in that**
the active substance is indirectly immobilized on the receptor by means of active substance-binding substances which additionally have at least one additional binding site for the receptor.

15. Use as claimed in claim 14,
**characterized in that**
the active substance-binding substances are selected from polylysine, dextran and protamine sulfate.

16. Use as claimed in claim 7,
**characterized in that**
the immobilization is achieved by forming a matrix inside the ghost.

17. Use as claimed in claim 16,
**characterized in that**
the matrix is formed inside the ghost by polymerization or/and copolymerization of monomers.

18. Use as claimed in claim 16 or 17,
**characterized in that**
the polymerization is started by increasing the temperature, by UV radiation or/and addition of initiators.

19. Use as claimed in claim 16 or 17,
**characterized in that**
an enzyme-catalysed polymerization is carried out.

20. Use as claimed in claim 19,
**characterized in that**
enzymes are used which catalyse the synthesis of polyhydroxyfatty acids, polysaccharides or polypeptides.

21. Use as claimed in claim 16,
**characterized in that**
the matrix is formed by the aggregation of substances capable of aggregation.

22. Use as claimed in one of the claims 4 to 21,
**characterized in that**
the ghosts contain heterologous surface molecules that are specific for target cells or target tissue.

23. Use as claimed in one of the claims 4 to 22 to produce an agent for the medical field.

24. Use as claimed in claim 23 to produce an agent for preventing or/and for combating diseases caused by pathogens, tumour diseases or autoimmune diseases.

25. Use as claimed in claim 23 or 24 to produce an agent for gene therapy.

26. Use as claimed in claim 23 or 24 to. produce an agent for nucleic acid vaccination.

27. Use as claimed in claim 23 to produce an agent for diagnostic purposes.

28. Use as claimed in one of the claims 23 to 27 to produce an agent,
wherein the ghosts are administered by the same route as that of the natural infection of the organism with the pathogen.

29. Use as claimed in one of the claims 4 to 22 in the agricultural field.

30. Use as claimed in one of the claims 4 to 29,
**characterized in that**
the ghosts contain several different active substances.

31. Use as claimed in one of the claims 4 to 30,
**characterized in that**
mixtures of ghosts each containing different active substances are used.

32. Use as claimed in one of the claims 4 to 30,
**characterized in that**
the ghosts are derived from gram-negative or/and gram-positive bacteria.

33. Use of bacterial ghosts to prepare a nucleic acid vaccine.

34. Use of bacterial ghosts as carrier or/and targeting vehicles for a nucleic acid vaccine.

35. Use as claimed in claim 33 or 34,
**characterized in that**
the nucleic acid packaged in the ghosts contains a sequence coding for the antigen to be expressed in operative linkage with expression control sequences.

36. Use as claimed in claim 35,
**characterized in that**
the nucleic acid additionally contains a bacterial origin of replication, a prokaryotic selection marker gene, a reporter gene or/and immunomodulatory sequences.

37. Use as claimed in one of the claims 33 to 36,
**characterized in that**
the ghosts contain several different antigen-encoding nucleic acids.

38. Use as claimed in one of the claims 33 to 37,
**characterized in that**
a homologous combination of bacterial ghosts and antigen-encoding nucleic acids is used.

39. Use as claimed in one of the claims 33 to 37,
**characterized in that**
a heterologous combination of bacterial ghosts and antigen-encoding nucleic acids is used.

40. Pharmaceutical or agricultural composition comprising a bacterial ghost as claimed in claim 2 or 3 containing an active substance packaged therein.

## Revendications

1. Procédé de préparation d'un hôte bactérien contenant une substance active englobant les étapes suivantes
(a) Mise à disposition d'hôtes bactériens et
(b) Mise en contact de l'hôte bactérien avec une substance active.

2. Hôte bactérien avec une substance active encapsulée dans celui-ci pouvant être obtenu avec le procédé selon revendication 1.

3. Hôte bactérien selon revendication 2,
**caractérisé en ce que**
la substance active est un acide nucléique.

4. Utilisation d'hôtes bactériens selon revendication 2 ou 3 pour le conditionnement des substances actives.

5. Utilisation d'hôtes bactériens selon revendication 2 ou 3 comme excipient de support ou/et de ciblage pour une substance active.

6. Utilisation selon revendication 4 ou 5,
**caractérisée en ce que**
la substance active est choisie parmi des substances actives sur le plan pharmacologique, des substances de marquage, des substances actives dans le domaine agricole et des colorants.

7. Utilisation selon l'une quelconque des revendications 4 à 6,
**caractérisée en ce que**,
la substance active est présente dans les hôtes bactériens sous forme immobilisée.

8. Utilisation selon revendication 7,
**caractérisée en ce que**,
l'immobilisation est réalisée au moyen d'interactions avec un récepteur localisé sur le côté intérieur de la membrane des hôtes.

9. Utilisation selon revendication 8,
**caractérisée en ce que**,
le récepteur est un polypeptide hétérologue intégré dans la membrane cytoplasmatique des hôtes.

10. Utilisation selon revendication 8 ou 9,
**caractérisée en ce que**,
le polypeptide hétérologue est un polypeptide de fusion contenant de la streptavidine ou de l'avidine

11. Utilisation selon l'une quelconque des revendications 7 à 10,
**caractérisée en ce que**,
il se produit une immobilisation directe de la substance active sur le récepteur.

12. Utilisation selon revendication 11,
**caractérisée en ce que**,
l'on utilise une substance active dérivée avec des groupes de liaison d'un récepteur.

13. Utilisation selon l'une quelconque des revendications 7 à 10,
**caractérisée en ce que**
il se produit une immobilisation indirecte de la substance active sur le récepteur.

14. Utilisation selon revendication 13,
**caractérisée en ce que**
l'immobilisation indirecte de la substance active sur le récepteur intervient au moyen de substances liant les substances actives, lesquelles possèdent par ailleurs au moins un point de liaison supplémentaire pour le récepteur.

15. Utilisation selon revendication 14,
**caractérisée en ce que**
les substances liant les substances actives sont choisies parmi la polylysine, le dextrane et le sulfate de protamine.

16. Utilisation selon revendication 7,
**caractérisée en ce que**
l'immobilisation intervient par formation d'une matrice à l'intérieur de l'hôte.

17. Utilisation selon revendication 16,
**caractérisée en ce que**
la matrice se forme par polymérisation ou/et copolymérisation de monomères à l'intérieur de l'hôte.

18. Utilisation selon revendication 16 ou 17,
**caractérisée en ce que**
l'on démarre la polymérisation en augmentant la température, en irradiant des rayons UV ou/et en ajoutant des initiateurs.

19. Utilisation selon revendication 16 ou 17,
**caractérisée en ce que**
il se produit une polymérisation catalysée par des enzymes.

20. Utilisation selon revendication 19,
**caractérisée en ce que**
l'on utilise des enzymes qui catalysent la synthèse des acides gras polyhydroxyliques, les polysaccharides et les polypeptides.

21. Utilisation selon revendication 16,
**caractérisée en ce que**
la matrice est formée par association de substances agrégables.

22. Utilisation selon l'une quelconque des revendications 4 à 21,
**caractérisée en ce que**
les hôtes contiennent des molécules de surface hétérologues, spécifiques aux cellules cibles ou aux tissus cibles.

23. Utilisation selon l'une quelconque des revendications 4 à 22 pour la préparation d'un agent pour le domaine médical.

24. Utilisation selon revendication 23 pour la préparation d'un agent destiné à la prévention ou/et à la lutte contre des maladies provoquées par des agents pathogènes, des pathologies tumorales ou des pathologies auto-immunes.

25. Utilisation selon revendication 23 ou 24 pour la préparation d'un agent pour la génothérapie.

26. Utilisation selon revendication 23 ou 24 pour la préparation d'un agent destiné à la vaccination à l'acide nucléique.

27. Utilisation selon revendication 23 pour la préparation d'un agent à des fins diagnostiques.

28. Utilisation selon l'une quelconque des revendications 23 à 27,
pour la préparation d'un agent, l'administration de l'hôte étant prévue par la même voie que l'infection naturelle de l'organisme par l'agent pathogène.

29. Utilisation selon l'une quelconque des revendications 4 à 22 dans le domaine agricole.

30. Utilisation selon l'une quelconque des revendications 4 à 29,
**caractérisée en ce que**
les hôtes contiennent plusieurs substances actives différentes.

31. Utilisation selon l'une quelconque des revendications 4 à 30,
**caractérisée en ce que**
on utilise des mélanges d'hôtes contenant chacun différentes substances actives.

32. Utilisation selon l'une quelconque des revendications 4 à 30,
**caractérisée en ce que**
les hôtes proviennent de bactéries Gram négatives ou/et Gram positives.

33. Utilisation d'hôtes bactériens pour la préparation de vaccins à l'acide nucléique.

34. Utilisation d'hôtes bactériens comme excipient de support ou/et de ciblage pour un vaccin à l'acide nucléique.

35. Utilisation selon revendication 33 ou 34,
**caractérisée en ce que**
l'acide nucléique conditionné dans les hôtes contient une séquence codante pour l'antigène à exprimer en liaison opérationnelle avec des séquences de contrôle de l'expression.

36. Utilisation selon revendication 35,
**caractérisée en ce que**
l'acide nucléique contient par ailleurs une origine de réplication bactérienne, un gène marqueur de sélection procaryotique, un gène reporteur ou/et des séquences immunomodulatoires.

37. Utilisation selon l'une quelconque des revendications 33 à 36,
**caractérisée en ce que**
les hôtes contiennent plusieurs acides nucléiques différents codant les antigènes.

38. Utilisation selon l'une quelconque des revendications 33 à 37,
**caractérisée en ce que**
l'on utilise une combinaison homologue d'hôtes bactériens et d'acides nucléiques codant les antigènes.

39. Utilisation selon l'une quelconque des revendications 33 à 37,
**caractérisée en ce que**
l'on utilise une combinaison hétérologue d'hôtes bactériens et d'acides nucléiques codant les antigènes.

40. Composition pharmaceutique ou agricole comprenant un hôte bactérien selon la revendication 2 ou 3 avec une substance active conditionnée dans celui-ci.
